Europäisches Patentamt

(19) European Patent Office

Office européen des brevets

(11) Publication number: **0 443 809 A2**

# (12) EUROPEAN PATENT APPLICATION

(21) Application number: **91301301.7**

(22) Date of filing: **19.02.91**

(51) Int. Cl.⁵: **A61F 2/16, A61L 27/00**

(30) Priority: **20.02.90 US 481970**

(43) Date of publication of application:
**28.08.91 Bulletin 91/35**

(84) Designated Contracting States:
**AT BE CH DE DK ES FR GB IT LI NL SE**

(71) Applicant: **IOPTEX RESEARCH INC.**
**15715 Arrow Highway**
**Irwindale, California 91706-2094 (US)**

(72) Inventor: **Colvin, Michael**
**25001 Pacific Coast Highway**
**Malibu, California 90265 (US)**
Inventor: **Barnes, Joanna Elizabeth**
**2264 Canterbury Avenue**
**Pomona, California 91768 (US)**

(74) Representative: **Cole, William Gwyn, Dr.**
**Smith & Nephew p l c Corporate Patents and**
**Trade Marks Dept. Gilston Park**
**Harlow Essex CM20 2RQ (GB)**

(54) **Coated intraocular lens and coatings therefor.**

(57) A coating material is produced useful for coating an intraocular lens having haptics secured thereto or integral therewith or for coating one or more of said haptics or for coating both which contains at least one substance to be released at a predetermined controlled rate into the human eye together with the coating material. The coating material is hydrophilic, compatible with the lens or haptics both onto which it is coated, is non-toxic to the human eye, and is biocompatible with the human eye. The coating is capable of delivering a desired effective amount of the at least one substance into the human eye over a predetermined period of time, together with a method of releasing a desired substance into the human eye by implanting into the human eye an intraocular lens having a coating as above described.

EP 0 443 809 A2

# COATED INTRAOCULAR LENS AND COATINGS THEREFOR

The present invention relates to coatings which are particularly useful for coating intraocular lenses (IOLs) which coating contains at least one substance to be released at a predetermined controlled rate into the human eye together with the coating itself. The substance to be released can be a therapeutic or diagnostic substance.

It is known in the art to coat lenses with various substances. It is also known in the art to topically apply various therapeutic and diagnostic substances direct into the eye.

The eye is a unique organ which differs from other organs of the body quite distinctively. It is protected physically by tough layers of sclera and the cornea and is anatomically isolated from the rest of the body by blood-eye barriers. Although the eye is a tough organ its delicate structures are subject to a number of diseases and disorders, for example, cataracts of the natural lens.

The opacification of the crystalline lens of the eye (cataracts) are quite common in the elderly. In 1987 there were over one million cataract operations performed in the United States alone. The majority of these operations include the implantation of an IOL. The acute complication rate with IOL implantation is quite low, around 0.5 - 1.0%. The types of complications which are associated with IOL implantation include infection such as potentially devastating infection endothalmitis, inflammation such as iritus or uveitus, capsular opacification of the posterior capsule, complement activation, and edema.

Most of these complications may be minimised or treated with medication. The common methods of treating ocular inflammation and infection associated with IOL implantation include topical application, perfusion, oral administration, and intravenous, subconjunctival and intraocular injection.

All of the above methods have inherent disadvantages associated with usage. Topical administration of medication is limited to drugs which can pass through the cornea. The cornea is a unique tissue which has an aqueous stroma sandwiched by two lipid layers ; the endothelium and the epithelium. As a result, a drug which is both hydrophilic and hydrophobic can penetrate the corneal tissue freely. On the other hand if the drug is purely polar or nonpolar it will not penetrate the corneal tissue effectively. If the drug does not pass freely, the therapeutic concentration of the drug is never reached within the eye. A major drawback to topical application is the short duration of drug action. Therefore, the application must be repeated in order to keep the level of drug in the eye at a therapeutic level. Similar arguments hold for ointments.

Oral or systemic administration delivers the drug to the eye through the bloom perfusing the ocular tissues. The amount of blood entering the eye from the blood in ocular vessels is limited by the blood-ocular barrier. The blood-ocular barrier results in extremely low ocular concentrations from systemically administered drugs. The barrier present a lipoidal barrier and the drugs appear to cross the barrier in proportion to their lipid solubility (Maurice et al Seals ML, ed. "Pharmacology of the Eye" New York : Springer-Verlag, 1984). The overall ocular concentration of drug delivered via this method depends on free drug serum concentration-time curve, the ability to cross the blood-ocular barrier, the effects of the active transport systems on the drug, and the presence of ocular inflammation (Lesar et al., Drug Intell. CLin. Pharm. 19 [1985]).

When drugs are administered topically, systemically, or by periocular injections the ocular penetration is usually poor. Intraocular injections are sometimes used to deliver drugs to the ocular structure, in particular, for the treatment of endothalmitus (Peyman et al., Trans. Am. Acad. Opthal. Otolar. 78, 862, [1974]).

However, in most cases intraocular injection is not a recommended practice because of the danger of complications.

Liu et al (Opthalmology 94, 9, 1155, [1987]) studied drug delivery to the eye in rabbit models by the intravitreal injection of liposomes encapsulated with trifluorothymidine. A similar study was conducted by Fishman et al (Invest. Opthalmol. Vis. Sci., 27, 1103 [1986]) where they studied the release rate of liposome encapsulated gentamicin injection intravitreally. However, there has not been any published work where these methods have been used to deliver drugs in conjunction with IOL implantation.

There has been extensive laboratory and clinical research in the use of vescoelastic materials in intraocular lens surgery (Polack et al. Opthalmology, 88, 425, [1981]). These observations have led to the use of viscoelastic material as an important adjunct to many ophthalmic operative procedures. In regard to IOL surgery viscoelastic material is injected into the eye commonly through the site of incision with a cannula. However, it has been shown that if the material is injected into the eye in a careless manner great damage may occur to the eye. Pape et al (Opthalmology, 88, 699 [1980]) have reported a tendency in the rise in the intraocular pressure (IOP) with the use of a viscoelastic, especially when excessive amounts were utilised.

The present invention represents a substantial advance in the art by virtue of a unique coating which may be placed on at least a portion of at least one surface of an IOL which is then subsequently implanted into the human eye.

In accordance with the present invention there is provided an intraocular lens comprising a lens body and optionally two haptics either attached to the lens body or formed integrally therewith and having a coating on at least a portion of at least one surface of said lens, which coating comprises a coating material and a releasable pharmacologically active agent in which said coating material is hydrophilic, compatible with the lens body or haptics onto which it is coated, and is non-toxic to and biocompatible with the human eye and which the pharmacologically active agent is present in a pharmacologially effective amount and is capable of being released at a predetermined rate from said coating.

The present invention also provides a coating composition for an intraocular lens comprising a coating material and a releasable pharmacologically active agent wherein said coating material is hydrophilic compatible with the portions of the lens to be coated and is non-toxic to and biocompatible with the human eye and wherein said pharmacologically effective amount and capable of being released at predetermined rate for said composition when coated onto said intraocular lens.

More particularly the present invention comprises a coating material useful for coating an intraocular lens having haptics secured thereto or integral therewith or for coating one or more of said haptics, or for coating both said intraocular lens and said haptics, which coating contains at least one substance to be released at a predetermined controlled rate into the human eye together with the coating material, is hydrophilic, compatible with the lens material or the haptic material or both materials onto which it is coated, is non-toxic to the human eye, and is biocompatible with the human eye, said coating being capable of delivering a desired effective amount of at least one said substance into the human eye over a predetermined period of time.

According to one embodiment, at least one substance is a therapeutic agent present in a therapeutically effective amount.

According to a further embodiment, at least one substance is diagnostic agent present in an effective amount.

Examples of such substances according to the present invention include : an anti-inflammatory agent present in an anti-inflammatory effective amount ; an anti-microbial agent present in an anti-microbially effective amount ; an anti-mycotic agent present in an anti-mycoticly effective amount ; an anti-viral agent present in an anti-virally effective amount ; an anti-fungal agent present in an anti-fungally effective amount ; and an immunosuppressive agent present in immonosuppressively effective amount.

According to another embodiment of the present invention suitable for coating materials include hydroxy propylmethyl cellulose, a combination of hydroxy propylmethyl cellulose and vinyl alcohol, and collagen. Preferably the hydroxy propylmethyl cellulose and vinyl alcohol are present in a ratio of 10 :90 to 90 :10.

According to a further embodiment, the present invention includes an intraocular lens wich comprises a lens body or a lens body and two haptics attached thereto or integral therewith, having on at least one surface of one, portion thereof, a coating material which is hydrophilic, compatible with the lens or the haptics or both onto which it is coated, is non-toxic to the human eye, and is biocompatible with the human eye which coating contains at least one substance to be released at a predetermined controlled rate into the human eye together with the coating material, said coating being capable of delivering a desired effective amount of said at least one substance into the human eye over a predetermined period of time.

Examples of suitable substances and coating materials are those described above.

According to a further embodiment, the coating comprises more than one layer of coating material.

According to a further embodiment, the coating is on at least a portion of the periphery of the lens.

According to a further embodiment, the coating is on at least one portion of one lens surface.

According to a further embodiment, the coating is on at least one of the haptics.

According to a further embodiment, the coating is on substantially all of one surface of the lens.

According to a further embodiment, the coating is on two different portions of the lens surface. In that case, two coatings may contain the same substance or each coating contains a different substance.

According to a further embodiment, the lens has a second coating containing the same or a different substance on at least one haptic. In that case the coatings may contain the same substance or each coating contains a different substance.

According to a further embodiment, each coating comprises at least one layer.

According to a further embodiment, the lens has a second coating on at least a portion of its other surface. In that case, each coating may contain the same substance or each coating contains a different substance.

According to a further embodiment, each coating comprises at least one layer.

The present invention also includes a method of releasing a desired substance into the human eye which comprises implanting into a human eye an intraocular lens having haptics secured thereto or integral therewith having on at least one portion of at least one surface thereof a coating material which contains at least one substance to be released at a predetermined controlled rate into the human eye together with the coating with the coating material, said coating material being hydrophilic, compatible with the lens or haptic or both onto which it is coated, non-toxic to the human eye, and biocompatible with the human eye, said coating being capable of delivering a desired effective amount of said at least one substance into the human eye over a predetermined period of time. The substances, coating materials, areas of coating and number of layers are as above described.

The following non-limitative examples more particularly illustrate the present invention.

## Example 1 - Preparation of Fluorescein

### Isothiocyanate

A mass (x) varying from 0.005 to 0.1 grams of Fluorescein Isothiocyanate (FITC) was added to 40 ml of ultrapure water. The mixture was fully dissolved by agitation by placing the vial containing the contents in a ultrasonic bath. The solution was then filtered with a 0.45 micron filter. The polymer solution was prepared by adding two grams of Hydroxypropyl Methyl Cellulose (M.W. 86,000) (HMPC) to forty ml of ultrapure water. The polymer was dissolved by repeated heating to 90°C and cooling to 15°C. Once the polymer was completely dissolved an equal volume of the FITC and the HPMC solutions were combined.

## Example 2 - HPMC/FITC Coated Lens

A sample of the product of Example 1 wherein X= 0.02 grams was aspirated into a syringe. Droplets were placed onto the convex side of an IOL until the optic was coated. Once the coating was uniform the lens was dried in a particulate free environment. The lens was then rotated and the process was repeated on the opposite side. The process was repeated so that both sides were double coated with a final coating weight of 0.006 grams. The IOL was implanted into the eye of a New Zealand albino rabbit in the posterior chamber using phacoemulsification endocapsular lensectomy implant technique. The release of fluorescein from the implant into the interior of the eye was followed measurements as a function of time. The release into the eye followed first order kinetics from $t_o$ to three hours after implantation. The total mass of fluorescein released into the eye was 60 µg.

## Example 3 - HPMC/FITC Coated Lens

A sample of Example 1 with X= 0.02 grams was aspirated into syringe. An IOL was coated, implanted, and the release of FITC was measured in a rabbit model in a similar manner to that described in Example 2. The mass of the final coating was 0.12 grams. The release into the eye followed $t_{1/12}$ order kinetics from $t_o$ to 80 minutes after implantation ; the release rate then fell off exponentially thereafter. The total mass of fluorescein released into the eye was 1.2mg.

## Example 4 - HPMC/FITC Coated Lens

A sample of Example 1 with X= 0.007g was used to coat an IOL as in Example 2, but was coated with two layers on one side of the lens only. The lens was implanted into the eye of a rabbit model, and the release kinetics followed with fluorophotometry as described in Example 2. The release of FITC followed zero order kinetics after implantation wich continued three hours thereafter. The total mass of FITC delivered intraocularly was 3.5 µg.

## Example 5 - HPMC/FITC Coated Lens

A sample of Example 1 with X= 0.007 g was used to coat an IOL using a syringe as described as in Example 2. The IOL was coated with two layers on one side of the lens only. The lens was implanted into the eye of a rabbit model, and the release kinetics followed with fluorophotometry as described in Example 2. The release of FITC followed zero order kinetics from $t_o$ to three hours. The total mass of FITC delivered in the eye was 3.5 µg.

## Example 6 - HPMC/FITC Extended Release Coated Lens

A coated lens with X= 0.0005 graphs was prepared as in Example 2. After drying the coated lens was placed into one ml of 1,6 diisocyanato-hexane for 2.5 hours. The lens was removed and dried. The release kinetics of FTIC from the IOL was followed by fluorescence spectrophotometry. The release kinetics followed first order kinetics from $t_o$ up to 50 hours, the total amount of FITC released was 0.5 µg.

## Example 7 - HPMC/Vancomycin Hydrochloride Coated Lens

A mass comprising 0.02 grams of Vancomycin Hydrochloride was dissolved into 40 ml of ultrapure water with agitation. A HMPC polymer solution was pared as described in Example 1. Equal volumes of the Vancomycin and HPMC solutions were combined with good mixing. An IOL was coated twice on both sides as described in Example 2, with complete drying. The release kinetics were followed using UV spectroscopy by placing the lens in 3 ml of saline at 37°C. The release of tetracycline followed $t_{1/12}$ kinetics from to up to one hour, the total amount of tetracycline released was 25 µg.

## Example 8 - HPMC/Tetracycline Hydrochloride Coated Lens

A mass comprising 0.02 g of tetracycline Hydrochloride dissolved in 40 ml of ultrapure water. A HPMC solution was-prepared as described in Example 1. Equal volumes of Tetracycline and HPMC solution were combined with good agitation. An IOL was coated with complete drying. The release kinetics were followed by UV spectroscope in Example 7. The release of tetracycline followed $t_{1/12}$ kinetics from $t_0$ up to one hour, the total amount of tetracycline released was 25 µg.

## Example 9 - HPMC/Hydrocortisone Coated Lens

A mass comprising 0.01 gram of hydrocortisone was dissolved into 20 ml of ultrapure water. A HMPC solution was prepared as described in Example 2. Equal volumes of hydrocortisone and HMPC solution were combined with good agitation. An IOL was coated twice on both sides with the mixture as described in Example 2 and with complete drying. The release kinetics were followed by UV spectroscopy as in Example 7. The release kinetics of hydrocortisone followed t1/12 kinetics from $t_0$ up to two hours, the total amount of hydrocortisone released was 2.2 µg.

## Example 10 - HPMC-Polyvinyl Alcohol/Methotrexate Coated Lens

A solution of HPMC and Poly vinyl alcohol was prepared in the following manner : 2g of HPMC (M.W. 86,000) and 1 g polyvinyl alcohol (M.W. 110,000) were added to 100 ml of ultrapure water. The polymers were dissolved by repeated heating to 90°C and cooling to 15°C until they were completely dissolved. 0.2 g of methotrexate was added to 2 mL of the polymer solution. An IOL was coated on both side with the mixture as described in Example 2 with complete drying between coatings. The release kinetics were followed by UV spectroscopy as described in Example 7. The release of methotrexate followed t1/12 kinetics from $t_0$ up to three hours, the total amount of methotrexate delivered was 8 mg.

## Example 11 - Collagen/Hydrocortisone Coated Lens

A mass comprising of 0.01 g of hydrocortisone was dissolved in 20 ml of ultrapure water. In parallel 0.01 g of collagen was dissolved into 20 ml of ultrapure water with low heat. Equal volumes of hydrocortisone and collagen were combined with good agitation. An IOL was coated once on one side only with the mixture. The release kinetics were followed by UV spectroscopy as described in Example 7. The release of hydrocortisone followed first order kinetics from $t_0$ up to six hours ; the total amount of hydrocortisone released was 2 µg.

## Example 12 - HPMC/Ibuprofen Coated Lens

A mass comprising 0.01 g of Ibuprofen was dissolved into 10 ml of ultrapure water. In parallel, a mass comprising 2 grams of hydroxypropyl methyl cellulose was dissolved into 100 ml of ultrapure water by repeated heating to 90°C and cooling to 15°C. To the Ibuprofen mixture was added 10 ml of the polymer solution. An IOL was coated once on one side only with the mixture using a syringe. The release kinetics were followed by UV spectroscopy as described in Example 7. The release of Ibuprofen followed first order kinetics from $t_0$ up to 8 hours ; the total amount of Ibuprofen released was 3 µg.

## Claims

1. In accordance with the present invention there is provided an intraocular lens comprising a lens body and optionally two haptics either attached to the lens body or formed integrally therewith and having a coating on at least a portion of at least one surface of said lens, which coating comprises a coating material and a releasable pharmacologically active agent in which said coating material is hydrophilic, compatible with the lens body or haptics onto which it is coated, and is non-toxic to and biocompatible with the human eye and which the pharmacologically active agent is present in a pharmacologially effective amount and is capable of being released at a predetermined rate from said coating.

2. A lens according to claim 1 wherein said pharmacologically active agent is a therepeutic agent present in a therapeutically effective amount.

3. A lens according to claim 1 wherein said pharmacologically active agent is a diagnostic agent present in an effective amount.

4. A lens according to claim 1 wherein said pharmacologically active agent is an anti-inflammatory agent present in an anti-inflammatory effective amount.

5. Coating material according to claim 1 wherein said pharmacologically active agent is a anti-microbial agent present in an anti-microbially effective amount.

6. A lens according to claim 1 wherein said pharmacologically active agent is an anti-mycotic agent present in an anti-mycoticly effective amount.

7. A lens according to claim 1 wherein said pharmacologically active agent is an anti-viral agent present in an anti-virally effective amount.

8. A lens according to claim 1 wherein said pharmacologically active agent is an anti-fungal agent present in an anti-fungally effective amount.

9. A lens according to claim 1 wherein said pharmacologically active agent is an imnonosuppressive agent present an immunosuppressively effective amount.

10. A lens according to any one of the preceding claims wherein the coating material is a hydroxy propylmethyl cellulose.

11. A lens according to claim 10 wherein the coating material is a combination of hydroxy propylmethyl cellulose and vinyl alcohol.

12. A lens according to claim 11 wherein the hydroxy propylmethyl cellulose and vinyl alcohol are present in a ratio of 10 :90 to 90 :10.

13. A lens according to any one of claims 1 to 9 wherein the coating material is collagen.

14. A lens according to any one of the preceding claims wherein the coating comprises more than one layer of coating material.

15. A lens according to any one of the preceding claims wherein the coating is on at least a portion of the periphery of the lens.

16. A lens according to any one of the preceding claims wherein the coating is on at least one portion of one lens surface.

17. A lens according to any one of the preceding claims wherein the coating is on at least one of the haptics.

16. A lens according to any one of the preceding claims wherein the coating is on substantially all of one surface of the lens.

19. A lens according to any of the preceding claims wherein the coating is on substantially on all of both surfaces of the lens.

20. A lens according to any one of the preceding claims wherein the coating is on two different portions of the lens surface.

21. A lens according to claim 20 wherein the two coatings contain the same pharmacologically active agent.

22. A lens according to claim 20 wherein each coating contains different pharmacologically active agents.

23. The present invention also provides a coating composition for an intraocular lens comprising a coating material and a releasable pharmacologically active agent wherein said coating material is hydrophilic compatible with the portions of the lens to be coated and is non-toxic to and biocompatible with the human eye and wherein said pharmacologically effective amount and capable of being released at predetermined rate for said composition when coated onto said intraocular lens.